(19) Europäisches Patentamt
European Patent Office
Office européen des brevets

(11) Veröffentlichungsnummer: **0 225 974**
**A2**

(12) # EUROPÄISCHE PATENTANMELDUNG

(21) Anmeldenummer: **86112164.8**

(22) Anmeldetag: **03.09.86**

(51) Int. Cl.$^4$: **C07D 201/04**

(30) Priorität: **02.11.85 DE 3538859**

(43) Veröffentlichungstag der Anmeldung:
**24.06.87 Patentblatt 87/26**

(84) Benannte Vertragsstaaten:
**CH DE FR GB IT LI**

(71) Anmelder: **HÜLS AKTIENGESELLSCHAFT**
**Patentabteilung / PB 15 - Postfach 13 20**
**D-4370 Marl 1(DE)**

(72) Erfinder: **Rademacher, Hans, Dr.**
**Badische Strasse 2**
**D-4370 Marl(DE)**
Erfinder: **Voges, Heinz-Werner, Dr.**
**Im Gorden 45**
**D-4270 Dorsten 21(DE)**

(54) **Verfahren zur Herstellung von Lactamen mit 8 bis 15 Kohlenstoffatomen aus den entsprechenden Oximen.**

(57) Gegenstand der Erfindung ist ein Verfahren zur Herstellung von Lactamen mit 8 bis 15 Kohlenstoffatomen durch Beckmannsche Umlagerung der entsprechenden Oxime in einer Lösung eines organischen Lösemittels mit Hilfe von 0,5 bis 10 Gewichtsprozent, bezogen auf das Oxim eines Säurederivates als Katalysator, wobei als Katalysator Anhydride organischer Sulfonsäuren oder von Schwefelsäurehalbestern oder von gemischten Anhydriden von organischen Sulfonsäuren und Schwefelsäurehalbestern verwendet werden.

EP 0 225 974 A2

Xerox Copy Centre

## Verfahren zur Herstellung von Lactamen mit 8 bis 15 Kohlenstoffatomen aus den entsprechenden Oximen

Die Herstellung von Lactamen durch Oximierung cycloaliphatischer Ketone und anschließende Beckmannsche Umlagerung ist bekannt. Die Beckmannsche Umlagerung wird in großtechnischem Maßstab üblicherweise mit Hilfe von konzentrierter Schwefelsäure oder Oleum vorgenommen. Bei dieser Arbeitsweise wird das Umlagerungsmittel in einer molaren Menge angewendet, die größer ist als die molare Menge des Oxims. Sie hat daher den Nachteil, daß erhebliche Mengen an verdünnter Schwefelsäure aufzubereiten oder zu beseitigen sind. Die Herstellung von Epsilon-Caprolactam aus Cyclohexanonoxim ist in Ullmanns Enzyklopädie der Technischen Chemie, Band 9, Seite 100 ff, 1975, die Herstellung von Laurinlactam aus Cyclododecanonoxim ist von K. Weissermel und H.-J. Arpe in dem Band Industrielle Organische Chemie, Verlag Chemie, Weinheim 1976, beschrieben.

Es ist auch bekannt, alicycliche Ketoxime mit 9 bis 14 Kohlenstoffatomen im Ring mit katalytischen Mengen von nur 0,5 bis 5 Gewichtsprozent, bezogen auf die Menge des Oxims, eines Säurechlorids, wie Thionylchlorid, Phosphoroxylchlorid, Phosphorpentachlorid, Benzolsulfochlorid umzulagern.

Da diese Katalysatoren in wesentlich geringeren Mengen verwendet werden können, bereitet ihre Abtrennung und Aufarbeitung naturgemäß nicht den Aufwand, wie er bei der Verwendung von Schwefelsäue oder Oleum als Umlagerungsmittel zu bewerkstelligen ist (DE-AS 25 34 538).

Diese Arbeitsweise ist jedoch mit einem erheblichen Nachteil verbunden. Das nach diesem Verfahren erhaltene Lactam enthält zwar geringe, jedoch nicht zu vernachlässigende Mengen an Chlor, in der Größenordnung von 100 bis 200 ppm. Derartig verunreinigte Lactame können nicht zur Herstellung der entsprechenden Polyamide verwendet werden. Insbesondere bei der Weiterverarbeitung der Polyamide zu Formkörpern verursachen die chlorhaltigen Verunreinigungen, insbesondere durch den gebildeten Chlorwasserstoff, bei den üblichen Verarbeitungstemperaturen Schäden an den Apparaturen und auch eine Schädigung der Polymeren.

Aufgabe der Erfindung ist es daher, Katalysatoren bereitzustellen, die in den bekannt niedrigen Mengen von 0,5 bis 10 Gewichtsprozent, bezogen auf die Oxime, verwendet werden können, die nach Ablauf der Umlagerung leicht zu entfernen sind und die vor allem frei von Chlor und anderen Halogenen in reaktionsfähiger Bindung sind, d. h., solche, welche keine halogenhaltigen Verunreinigungen in dem Lactam hinterlassen. Es besteht demnach die Aufgabe, halogenfreie Lactame herzustellen, die geeignet sind zur problemlosen Herstellung von Polyamiden und der daraus herzustellenden Formkörper.

Die Lösung der Aufgabe gelingt, wenn man als Katalysatoren Sulfonsäureanhydride, Anhydride von Schwefelsäurehalbestern oder gemischte Anhydride von Sulfonsäuren und Schwefelsäurehalbestern verwendet.

Geeignete Sulfonsäureanhydride sind solche von Alkyl-, Halogenalkyl-, Aryl-oder Aralkylsulfonsäuren der allgemeinen Formel

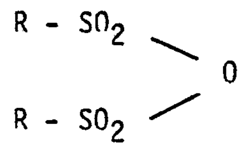

R bedeutet einen Alkylrest mit 1 bis 6 Kohlenstoffatomen, insbesondere 1 bis 2 Kohlenstoffatomen, oder einen Halogenalkylrest mit 1 bis 6, insbesondere 1 bis 2 Kohlenstoffatomen, wobei die Wasserstoffatome ganz oder teilweise durch Halogen ersetzt sein können.

Als Arylreste können Phenyl, Naphthyl odersubstituiertes Phenyl oder substituiertes Naphthyl fungieren und als Aralkylreste solche, die eine oder mehrere Alkylgruppen mit 1 bis 6, insbesondere 1 bis 2, Kohlenstoffatome enthalten.

Geeignete Sulfonsäureanhydride mit Alkylresten sind z. B. n-Propansulfonsäureanhydrid, Butansulfonsäueanhydrid oder Hexansulfonsäureanhydrid.

Insbesondere geeignet sind Methansulfonsäureanhydrid und Ethansulfonsäureanhydrid.

Als Sulfonsäureanhydride mit Halogenalkylresten seien genannt Chlormethansulfonsäureanhydrid, Fluormethansulfonsäueanhydrid, insbesondere Trichlormethansulfonsäureanhydrid und Trifluormethansulfonsäureanhydrid.

Als Sulfonsäureanhydride aromatischer Sulfonsäuren können verwendet werden Toluolsulfonsäureanhydrid, o-, m-, p-Chlorbenzolsulfonsäureanhydrid, o-, m-, p-Nitrobenzolsulfonsäureanhydrid, insbesondere Benzolsulfonsäureanhydrid und Naphthalinsulfonsäureanhydrid.

Die Sulfonsäureanhydride sind nach an sich bekannten Verfahren zugänglich (Organic Synthesis Coll. Vol. IV, Seite 940 (1963). Vorteilhaft können die Sulfosnsäureanhydride auch als Reaktionslösung eingesetzt werden, sofern sie zuvor von halogenhaltigen Reaktionspartnern, wie z. B. Thionylchlorid befreit sind.

Anhydride von Schwefelsäurehalbestern gehorchen der allgemeinen Formel

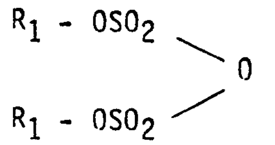 ,

wobei die beiden Reste $R_1$ auch zu einem Ring geschlossen sein können. $R_1$ kann ebenfalls einen Alkyl-oder Halogenalkylrest darstellen, wie er zuvor bei den Sulfonsäureanhydriden beschrieben ist, nämlich Alkyl mit 1 bis 6 Kohlenstoffatomen, insbesondere 1 Kohlenstoffatom oder Halogenalkyl mit 1 bis 6 Kohlenstoffatomen, insbesondere 1 Kohlenstoffatom, wobei die Wasserstoffatome ganz oder teilweise durch Halogen ersetzt sein können.

Geeignete Schwefelsäurehalbesteranhydride mit Alkylresten sind z. B. Diethylpyrosulfat, Dipropylpyrosulfat, Dibutylpyrosulfat, insbesondere Dimethylpyrosulfat. Als Schwefelsäurehalbesteranhydride mit Halogenalkylresten seien genannt Bischlormethylpyrosulfat, Bisfluormethyl pyrosulfat und Bisdichlormethylpyrosulfat, insbesondere Bistrifluormethylpyrosulfat und bistrichlormethylpyrosulfat.

Die Anhydride der Schwefelsäurehalbester können nach bekannten Verfahren erhalten werden (Matschinskaja, Below und Ussow, Z. obsc. Chim. 17, (1947) 2295; Chemical Abstracts 1948, 4918)

Gemischte Anhydride von Sulfonsäuren und Schwefelsäurehalbestern gehorchen der allgemeinen Formel

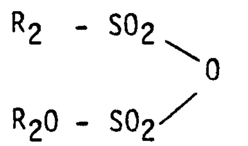 ,

wobei die Reste $R_2$ gleich oder verschieden oder zu einem Ring geschlossen sein können. $R_2$ kann ebenfalls einen Alkyl-, Halogenalkyl-oder aromatischen Rest darstellen, nämlich Alkyl mit 1 bis 6, insbesondee 1 Kohlenstoffatom oder Halogenalkyl mit 1 bis 6, insbesondere 1 Kohlenstoffatom, wobei die Wasseerstoffatome ganz oder teilweise durch Halogen ersetzt sein können. Als Arylreste können Phenyl, Naphthyl, substituiertes Phenyl oder substituiertes Naphthyl fungieren und als Aralkylreste solche, die eine oder mehrere Alkylgruppen mit 1 bis 6, insbesondere 1 Kohlenstoffatom enthalten. Geeignete Anhydride mit Alkylresten sind z. B. die Anhydride der Methansulfonsäure mit Schwefelsäuremonoethyl-, insbesondere -monomethylester. Geeignete Anhydride mit Halogenalkylresten sind z. B. die Anhydride der Trifluormethansulfonsäure mit Schwefelsäuremonomethyl-, -chlormethyl-und -trichlormethylester. Geeignete Anhydride mit Arylresten sind z. B. die Anhydride der Benzolsulfonsäure mit Schwefelsäuremonomethyl-, -trifluormethyl-, -ethyl-und -butylestern. Zu den cyclischen Verbindungen gehört das Carbylsulfat.

Die Herstellung der gemischten Anhydride kann z. B. nach dem Verfahren der GB-PS 666 154 erfolgen.

Besonders geeignet sind die Sulfonsäureanhydride, weil sie technisch am leichtesten zugänglich sind. Insbesondere sind sie auch deshalb geeignet, weil sie in sehr geringen Mengen einsetzbar und außerdem wohlfeil zu erhalten sind.

Die als Katalysatoren verwendeten Anhydride werden in Mengen von 0,5 bis 10 Gewichtsprozent, vorzugsweise in Mengen von 0,5 bis 5, und insbesondere in Mengen von 1 bis 3 Gewichtsprozent, bezogen auf die eingesetzten Oxime, verwendet.

Die Oxime cycloaliphatischer Ketone mit 8 bis 15 Kohlenstoffatomen können eingesetzt werden, vorteilhaft werden die der höhergliedrigen Ketone mit 10 bis 14 Kohlenstoffatomen eingesetzt, z. B. Cyclodecanonoxim, Cycloundecanonoxim, Cyclododecanonoxim, insbesondere Cycloundecanonoxim und Cyclododecanonoxim.

Die Umlagerung wird in organischen Lösemitteln vorgenommen. Geeignete Lösemittel sind solche, welche bei den Reaktionsbedingungen sowohl das Oxim als auch das Lactam hinreichend lösen, einen entsprechend hohen Siedepunkt besitzen und im Laufe der Umlagerung nicht verändert werden. Geeignet sind demnach üblicherweise sowohl aliphatische als auch cycloaliphatische oder aromatische Kohlenwasserstoffe.

Insbesondere sind geeignet Toluol, Isopropylcyclohexan. Derartige Lösemittel erlauben es, die Reaktionswärme durch Siedekühlung bei Normaldruck oder vermindertem Druck abzuführen.

Die Reaktionstemperatur liegt im allgemeinen bei 60 bis 140 °C, insbesondere bei 80 bis 120 °C. Die Reaktion läuft sehr schnell ab, im allgemeinen im Bereich von 1 bis 20, insbesondere von 1 bis 10 Minuten.

Die Reaktion wird im allgemeinen derart vorgenommen, daß man die vorerwähnte Oximlösung mit dem Katalysator versetzt. Zweckmäßigerweise führt man den Katalysator ebenfalls als Lösung oder Dispersion in dem gleichen Lösemittel zu. Die Reaktion setzt üblicherweise unter starker Wärmeentwicklung ein. Die Reaktionswärme wird üblicherweise durch Siedekühlung am Rückfluß abgeführt. Falls erforderlich, kann zusätzlich oder gegebenenfalls auch anstelle der Siedekühlung eine andere Kühlmethode durch Wärmeaustausch angewendet werden.

Das Ende der Umlagerung ist erkennbar am Absinken der Temperatur.

Die noch warme Reaktionslösung wird mit Wasser bis zur Neutralität gewaschen. Gegebenenfalls kann auch eine Nachbehandlung mit wäßrigen alkalischen Mitteln, wie z. B. einer Alkalihydroxidlösung, angeschlossen werden.

Aus der vom Katalysator befreiten Lösung wird das Lactam in üblicher Weise durch Destillation oder Kristallisation gewonnen. Falls zweckmäßig, kann bei der fraktionierten Destillation der Lactamlösung auch mit sogenannten Zwischensiedern gearbeitet werden, analog DE-B-15 45 696 bzw. DE-OS 17 95 575.

Vergleichsbeispiel (nach DE-AS 25 34 538)

Man löst in einem Kolben, der mit einem Thermometer, einem Rührer und einem Rückflußkühler versehen ist, 40 g Cyclododecanonoxim in 160 g Toluol und fügt zu der auf 95 °C erwärmten Lösung 0,8 g Thionylchlorid hinzu. Schon nach kurzer Zeit tritt die Umlagerungsreaktion ein, was man daran erkennt, daß das Reaktionsgemisch aufgrund der starken Wärmeentwicklung unter Rückfluß siedet. Nach fünf Minuten ist die Reaktion beendet, erkennbar am Nachlassen des Siedens und schließlich am Absinken der Temperatur. Eine dem Reaktionsgemisch entnommene Probe wird, z. B. mit Hilfe der Gaschromatografie, analysiert. Es zeigt sich, daß die Probe kein Oxim mehr enthält, d. h., daß der Oximumsatz 100 % beträgt. Die Laurinlactamkonzentration in der Probe entspricht einer Laurinlactamausbeute von 98 %.

Die Reaktionslösung wird nach Abkühlen auf 90 °C mehrmals gewaschen, bis die Waschwässer neutrale Reaktionen zeigen. Die Hälfte der gewaschenen Reaktionslösung läßt man bis auf Raumtemperatur abkühlen. Das dabei auskristallisierende Lactam wird isoliert und nochmals aus Toluol umkristallisiert. Das so erhaltene Lactam hat einen Chlorgehalt von 110 ppm. Die andere Hälfte der neutral gewaschenen Reaktionslösung wird durch Destillation aufgearbeitet. Dabei wird zunächst bei Normaldruck Toluol zusammen mit den kleinen Anteilen gelösten Wassers abdestilliert, danach bei einem Druck von 500 Pa das Lactam. Der Chlorgehalt in dem solcherart gewonnenen Lactam (Hauptfraktion) beträgt mindestens 100 ppm und kann bis zu 200 ppm betragen.

Beispiel 1

In einem Kolben, der mit einem Thermometer, einem Rührer und einem Rückflußkühler versehen ist, löst man 40 g Cyclododecanonoxim in 160 g Toluol und fügt zu der auf 95 °C erwärmten Lösung 0,8 g Benzolsulfonsäureanhydrid, gelöst in 10 g Toluol, hinzu. Sofort ist eine starke Wärmeentwicklung zu beobachten, so daß die Lösung unter Rückfluß siedet. Nach fünf Minuten ist die Reaktion beendet, erkennbar am Absinken der Temperatur. Die gaschromatografische Analyse der Reaktionslösung ergibt einen Oximumsatz von 100 % und eine Lactamausbeutevon über 99 %. Die noch warme Reaktionslösung wird zur Entfernung des Katalysaors bis zur Neutralität mit warmem Wasser gewaschen. Das durch Destillation gereinigte Laurinlactam ist frei von chlorhaltigen Verunreinigungen.

Beispiele 2 bis 11

Analog Beispiel 1 werden 40 g Cyclododecanonoxim in 200 ml Isopropylcyclohexan gelöst und bei 100 °C mit Art und Menge des Katalysators gelöst in ca. 10 ml Isopropylcyclohexan versetzt, wie in der Tabelle angegeben. Die durch Destillation gereinigten Lactame sind halogenfrei.

Beispiel 12

Wie in Beispiel 1 werden 40 g Cyclodecanonoxim in 200 ml Isopropylcyclohexan gelöst und bei 100 °C mit 0,8 g Benzolsulfonsäureanhyrid, gelöst in 10 ml Isopropylcyclohexan, versetzt. Laut Gaschromatografie wird ein Oximumsatz von 100 % und eine Decanolactamausbeute von 94,7 % erreicht. Das nach der Wasserwäsche durch Destillation erhaltene Decanolactam ist halogenfrei.

Beispiel 13

Analog Beispiel 1 werden 40 g Cycloundecanonoxim in 200 ml Isopropylcyclohexan gelöst und mit 0,8 g Benzolsulfonsäureanhydrid umgelagert. Laut Gaschromatografie wird bei vollständigem Oximumsatz eine Undecanolactamausbeute von 97,6 % erreicht. Das nach der Wasserwäsche durch Destillation erhaltene Undecanolactam ist halogenfrei.

Beispiel 14 (kontinuierliche Arbeitsweise)

Durch ein auf 100 °C thermostatisiertes Reaktionsrohr von 4000 mm Länge und mit einem Durchmesser von 10 mm werden stündlich 2,5 l (= 2 kg) einer 100 °C heißen Lösung von 25 Gewichtsprozent Cyclododecanonoxim in Isopropylcyclohexan sowie 47 ml einer heißen Lösung von 20 Gewichtsprozent Benzolsulfonsäureanhydrid in Isopropylcyclohexan (entsprechend 1,5 Gewichtsprozent Katalysator, bezogen auf Oxim) gepumpt. Der Reaktoraustrag wird in einer Rührkesselkaskade kontinuierlich zuerst mit wäßriger Natronlauge und sodann zweimal mit Wasser gewaschen. Man erhält pro Stunde 2,48 l Produktlösung, die 480 g Laurinlactam = 96 % Ausbeute enthält. Oxim läßt sich gaschromatografisch nicht nachweisen (< 0,1 %). Nach Entfernung des Lösungsmittels wird das Lactam destilliert. Es ist frei von chlorhaltigen Verunreinigungen.

Tabelle

| Beispiel | Katalysator | Menge Gew.-% bez. Oxim | Reaktionszeit Minuten | Oximumsatz % | Lactamselektivität °C |
|---|---|---|---|---|---|
| 2 | Methansulfonsäureanhyrid | 4 | 3 | 99,1 | 98,5 |
| 3 | Trifluormethansulfonsäureanhydrid | 3 | 3 | 100 | 99,2 |
| 4 | Benzolsulfonsäureanhydrid | 2 | 2 | 99,9 | 99,2 |
| 5 | p-Toluolsulfonsäureanhydrid | 10 | 10 | 85,1 | 90,9 |
| 6 | p-Chlorbenzolsulfonsäureanhydrid | 2 | 5 | 99,7 | 99,1 |
| 7 | p-Nitrobenzolsulfonsäureanhydrid | 0,5 | 5 | 97,3 | 98,5 |
| 8 | Dimethylpyrosulfat | 3 | 3 | 99,7 | 97,9 |
| 9 | 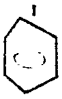$-SO_2-O-SO_2-O-nC_4H_9$ | 5 | 3 | 100 | 98,3 |
| 10 | $CF_3-SO_2-O-SO_2-O-C_{12}H_{25}$ | 5 | 5 | 100 | 98.6 |
| 11 | Carbylsulfat | 10 | 5 | 97,3 | 98,4 |

**Ansprüche**

1. Verfahren zur Herstellung von Lactamen mit 8 bis 15 Kohlenstoffatomen durch Beckmannsche Umlagerung der entsprechenden cyclischen Oxime mit 0,5 bis 10 Gewichtsprozent, bezogen auf das Oxim, eines Säurechlorids als Katalysator bei Temperaturen bis 140 °C in einer Lösung eines organischen

Lösemittels,
dadurch gekennzeichnet,
daß man als Katalysator Anhydride von organischen Sulfonsäuren oder von Schwefelsäurehalbestern oder gemischte Anhydride von organischen Sulfonsäuren und Schwefelsäurehalbestern verwendet.

2. Verfahren nach Anspruch 1,
dadurch gekennzeichnet,
daß man die Anhydride in Mengen von 1 bis 3 Gewichtsprozent, bezogen auf die Oxime, verwendet.

3. Verfahren nach den Ansprüchen 1 und 2,
dadurch gekennzeichnet,
daß man als Anhydrid Benzolsulfonsäureanhydrid verwendet.

4. Verfahren nach den Ansprüchen 1 und 2,
dadurch gekennzeichnet,
daß man als Anhydrid Methansulfonsäureanhydrid verwendet.

5. Verwendung der nach den Ansprüchen 1 bis 4 erhaltenen Lactame zur Herstellung der entsprechenden Polyamide, Polyesteramide oder Polyetheresteramide.